# EUROPÄISCHE PATENTANMELDUNG

(11) **EP 2 995 316 A1**
(43) Veröffentlichungstag der Anmeldung: **16.03.2016**
(21) Anmeldenummer: 14184416.7
(22) Anmeldetag: 11.09.2014
(51) Int. Cl.: A61K 41/00

(54) **Verfahren zur Bindung von Wirkagenzien an aktivierte Eigenblut-Nosoden und Vorrichtung zur Durchführung des Verfahrens**

(71) Anmelder: YE.NO Awareness B.V., 1017 BS Amsterdam (NL)
(72) Erfinder: Klein, Claudia Bettina, 71522 Backnang (DE)
(74) Vertreter: Samson & Partner Patentanwälte mbB

(57) **Zusammenfassung**

Eine Verfahren zur Bindung einer Wirksubstanz oder eines Wirkagens an eine aktivierte Eigenblut-Nosode umfasst a) das Lösen von Blut eines Patienten in einem wässrigen oder wässrig/ethanolischen Medium oder Verreiben von Blut eines Patienten mit einem nach HAB für Globuli zugelassenen Trägerstoff zum Erhalt einer ersten Mischung; b) das Aktivieren der ersten Mischung durch Einwirkenlassen von Magnetpulsen mit Frequenzen der Magnetfeldperioden innerhalb eines Bereich von etwa 0,01 bis etwa 20.0000 Hz und Feldstärken von maximal 50 µT auf die erste Mischung; c) die Zugabe einer bzw. eines oder mehrerer Wirksubstanzen und/oder Wirkagenzien zu der aktivierten ersten Mischung zum Erhalt einer zweiten Mischung; d) die Verschüttelung der zweiten Mischung durch mechanische Einwirkung, wobei die Schritte c) und d) und fortwährender Einwirkung der Magnetpulse vorgenommen werden und wobei die Schritte c) und d) einmal oder mehrmals wiederholt werden können; und e) das Aktivieren der verschüttelten zweiten Mischung durch weiteres fortwährendes Einwirkenlassen der Magnetpulse und Einstrahlen sichtbaren, von LEDs erzeugten Lichts wechselnder Farbe in die verschüttelte zweite Mischung, wodurch eine Steigerung der Bindungsfähigkeit des Human-Serumalbumin (HSA) in dem Blut an die Wirksubstanz(en) und/oder an zumindest Teile des Wirkagens bzw. der Wirkagenzien bewirkt wird. Eine Vorrichtung zur Durchführung des Verfahrens wird ebenfalls beschrieben.

## Beschreibung

### Gebiet der Erfindung

Die Erfindung betrifft ein Verfahren zur Bindung von Wirkagenzien an aktivierte Eigenblut-Nosoden sowie eine Vorrichtung zur Durchführung des Verfahrens.

### Hintergrund der Erfindung

Die Eigenbluttherapie ist mittlerweile nicht zuletzt dadurch, dass sie auf der Liste der verbotenen Dopingmittel steht, breiten Bevölkerungskreisen bekannt. Dabei wird dem Patienten Blut entnommen und an einer anderen Körperstelle dem Patienten wieder injiziert. Dabei gibt es auch Varianten, wie Bestrahlen des Bluts mit UV-C-Licht, Anreicherung des Bluts mit einem Ozon-Sauerstoff-Gemisch oder Zugabe von Nosoden, anderen homöopathischen Präparaten oder Immunstimulantien wie Echinacea, vor der Reinjektion des Bluts in den Patienten.

Weniger bekannt sind die sogenannten Eigenblut-Nosoden (auch als Eigenbluttherapie nach Imhäuser bezeichnet). Zu ihrer Herstellung wird ein Tropfen Blut aus der Fingerkuppe oder dem Ohrläppchen meist mit wässrigem Ethanol (z.B. 10 oder 20 ml) verdünnt oder auch mit beispielsweise Saccharose oder Xylitol verrieben und zu Globuli verarbeitet und die resultierende Dispersion bzw. die Globuli werden dem Patienten oral in Dosierungen von mehreren Tropfen bzw. pro Tag zurück verbreicht. Diese erste Verdünnung kann gemäß den Prinzipien der Homöopathie weiter verdünnt werden, sowohl in Form einer Lösung als auch in Form von Globuli. Bevorzugte Anwendung finden die Eigenblut-Nosoden z.B: bei allergischen und Hauterkrankungen sowie bei rezidivierenden Infektionskrankheiten. Als Wirkmechanismus wird eine unspezifische Umstimmungsreaktion vermutet.

Blut setzt sich bekanntermaßen aus Erythrozyten, Leukozyten, Thrombozyten und Blutplasma zusammen, wobei das letztgenannte zu etwa 90% aus Wasser und zu etwa 10% aus darin gelösten Stoffen, vor allem Salzen und Puffern (Säuren/Basen) sowie Proteinen (z.B. Albumin und Gerinnungsfaktoren), besteht.

Das wichtige Protein Albumin (oder Human-Serumalbumin (HSA)) dient in erster Linie zur Aufrechterhaltung des osmotischen Drucks des Blutes sowie zum Transport von wasserunlöslichen Substanzen im Blut. Ferner wirkt es desaggregierend auf Erythrozyten und Thrombozyten. HSA ist ein amphoteres Protein mit einer Molekülmasse von etwa 66.470 Da. Es ist von elliptischer Gestalt mit einem relativ harten Kern und einer flexiblen Oberflächenstruktur (Münnemann, Kerstin; Hinderberger, Dariush; Research Report from Webservice 2011 - Max-Planck-Institut for Polymer Research (http://www.mpip-Mainz.mpg.de/20689/research_report_1173941?c=22413)).

In der Medizin ist es seit einiger Zeit bekannt, dass die Bindung von Arzneistoffen an HSA erhebliche Vorteile mit sich bringen kann, z.B. eine Verringerung der erforderlichen Wirkstoffmenge, weniger Nebenwirkungen und ein gezielterer Transport des Wirkstoffs an den Ort des Krankheitsgeschehens. Die Bindung des Wirkstoffes an das HSA erfolgte dabei entweder durch kovalente Bindung (z.B. Methotrexat-HSA oder Aminopterin-HSA) oder im Fall von nab (nanoparticle albumin-bound)-Paclitaxel durch Hochdruckhomogenisierung von amorphem Paclitaxel in Anwesenheit von HSA zu einer kolloidalen Nanopartikel-Suspension.

Gewöhnliche Nosoden (also keine Eigenblut-Nosoden), der Namen sich aus dem griechischen Wort "nosos" ("Krankheit") ableitet, werden gemäß Vorschriften des HAB (Homöopathischen Arzneibuchs) aus (stark abgeschwächten oder abgetöteten) Krankheitserregern, pathologischem Material wie Blut eines Erkrankten, Eiter oder Zellen aus Organen, z.B. Krebszellen, oder Körpersekreten -Exkreten einschließlich beispielsweise Hormonen hergestellt (es sind derzeit mehr als 2000 Nosoden im Handel erhältlich). In der Nosode ist das Prinzip der Impfung (Anregung der Selbstheilungskräfte des Körpers, indem man ihn einem abgeschwächten Krankheitsreiz aussetzt) mit dem Prinzip der Homöopathie verbunden. Der Begriff "Nosode" wurde um 1830 von dem amerikanischen Arzt Constantin Hering geprägt. Das Prinzip ist aber bereits seit dem Altertum bekannt. Bereits um 800 v Chr. verwendeten die Chinesen verdünnte Pockensekrete, die zur Vorbeugung gegen die Krankheit unter die Haut geritzt wurden. Auch Hippokrates lehrte "Übles mit Üblem zu heilen", und der britische Philosoph und Arzt Robert Fludd beschrieb die Behandlung Schwindsüchtiger mit Verdünnungen ihres Auswurfs.

Ziel der Erfindung war es, die Vorteile einer Eigenblut-Nosode mit den Vorteilen der Bindung eines Wirkstoffes oder Wirkagens an HSA zu verbinden, d.h. das in der Eigenblut-Nosode vorliegende HSA mit einem Wirkstoff oder Wirkagens zu beladen, und zwar auf effektive, aber möglichst schonende Weise.

### Zusammenfassung der Erfindung

Die Erfindung betrifft ein Verfahren zur Bindung einer Wirksubstanz oder eines Wirkagens an eine aktivierte Eigenblut-Nosode, umfassend:
a) Lösen von Blut eines Patienten in wässrigen oder wässrig/ethanolischen Medium oder Verreiben von Blut eines Patienten mit einem nach HAB für Globuli zugelassenen Trägerstoff zum Erhalt einer ersten Mischung;
b) Aktivieren der ersten Mischung durch Einwirkenlassen von Magnetpulsen mit Frequenzen der Magnetfeldperioden innerhalb eines Bereichs von etwa 0,01 bis etwa 20.0000 Hz und Feldstärken von maximal 50 µT auf die erste Mischung zum Erhalt einer aktivierten ersten Mischung;
c) Zugabe einer bzw. eines oder mehrerer Wirksubstanzen und/oder Wirkagenzien zu der aktivierten ersten Mischung zum Erhalt einer zweiten Mischung;
d) Verschüttelung der zweiten Mischung durch mechanische Einwirkung, wobei die Schritte c) und d) und fortwährender Einwirkung der Magnetpulse vorgenommen werden und wobei die Schritte c) und d) einmal oder mehrmals wiederholt werden können, wodurch man eine verschüttelte zweite Mischung erhält; und
e) Aktivieren der verschüttelten zweiten Mischung durch weiteres fortwährendes Einwirkenlassen der Magnetpulse und Einstrahlen sichtbaren, von LEDs erzeugten Lichts wechselnder Farbe in die verschüttelte zweite Mischung, wodurch eine Steigerung der Bindungsfähigkeit des Human-Serumalbumin (HSA) in dem Blut an die Wirksubstanz(en) und/oder an zumindest Teile des Wirkagens bzw. der Wirkagenzien bewirkt wird und eine modifizierte Eigenblut-Nosode erhalten wird..

Ferner betrifft die Erfindung eine Vorrichtung zur Durchführung des erfindungsgemäßen Verfahrens, umfassend:
1) ein verschließbares Gehäuse,
2) einen in dem Gehäuse angeordneten Probenhalter, der ein transparentes Gefäß halten kann, in dem die erste Mischung von Schritt a) oder b) hergestellt wird oder enthalten ist,
3) eine Einrichtung, die wiederholt eine mechanische Erschütterung und/oder Translationsbewegung des Gefäßes bewirken kann,
4) eine Magnetspule, die auf das Gefäß gerichtete Magnetpulse mit Frequenzen der Magnetfeldperioden im Bereich von etwa 0,01 bis etwa 20.0000 Hz und Feldstärken von maximal 50 µT erzeugen kann; und
5) eine Einrichtung, die LEDs umfasst, welche sichtbares Licht mit mindestens zwei Farben erzeugen und das erzeugte Licht in das Gefäß einstrahlen lassen können.

### Kurze Beschreibung der Zeichnung

Fig. 1 zeigt eine schematische Schnittansicht entlang der Linie a-a von Fig. 2 einer Ausführungsform einer erfindungsgemäßen Vorrichtung.
Fig. 2 zeigt eine schematische Ansicht von oben der Vorrichtung von Fig. 1

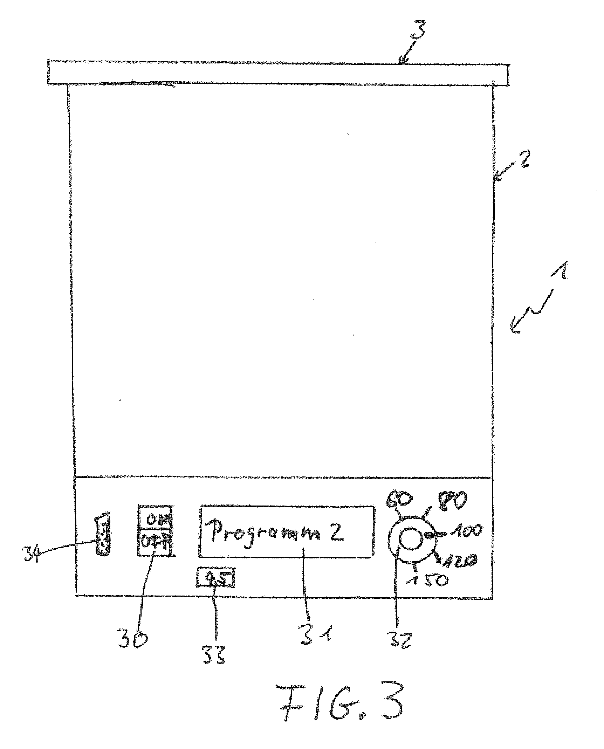

Fig. 3 zeigt eine Draufsicht von vorne der Vorrichtung von Fig. 1.

### Detaillierte Beschreibung

Die Eigenblut-Nosode, die in der Erfindung verwendet wird, ist vorzugsweise eine Suspension von einem Tropfen bis 3 ml, bevorzugt 2 ml Blut in 0,5 ml bis 20 ml, bevorzugt 1 ml bis 10 ml 40 bis 85 volumenprozentigem Ethanol, z. B. 0,5 ml Blut in 2 ml 70%igem Ethanol oder 2 ml Blut in 5 ml 70%-igem Ethanol.. Es können aber bei Alkohol-Unverträglichkeit des Patienten und bei Kindern auch physiologische Kochsalzlösung (0,9%ige NaCl-Lösung) oder übliche feste Globuli-Trägersubstanzen, wie Saccharose oder Xylitol, zur Verdünnung verwendet werden.

Diese Eigenblut-Nosode wird durch Magnetpulse innerhalb eines Frequenzbereichs von 0,01 bis 20.000 Hz und einer Magnetfeldstärke bis maximal 50 µT aktiviert. Die Pulse sind bevorzugt sogenannte Nadelpulse und weisen z.B. eine Sägezahnform auf. Häufig werden Nadelpuls-Pakete ähnlicher Form, aber mit variierender Feldstärke verwendet, z.B. können mehrere aufeinander folgende Pulspakete ähnlicher Form, aber mit variierender Feldstärke über eine Zeitraum von z.B. etwa 10 bis etwa 20 µs und einer Pause zwischen den Pulspaketen von etwa 55 bis etwa 75 µs verwendet werden. Geeignete Magnetpulse werden zum Beispiel von einem vita-life®-R-Magnetstab (erhältlich von VITA LIFE HandelsgmbH, Gewerbepark1, 9220, Velden-Lind, Österreich) geliefert. Die erste Aktivierung erfolgt bevorzugt über einen Zeitraum von etwa 0,5 bis etwa 10 Minuten, z.B. über etwa 1 oder 2 bis etwa 5 Minuten.

Es ist bekannt, dass pulsierende Magnetfelder mit geeigneter Frequenz Auswirkungen auf Blut haben. Für den Fall der eisenhaltigen Erythrozyten ist dies gut dokumentiert, aber auch die anderen ladungshaltigen oder polarisierbaren Blutkomponenten werden von den Magnetfeldern beeinflusst. Es wird angenommen, dass im Fall des HSA mit seinen vielen positiv und negativ geladenen Molekülteilen die Konformation des Moleküls durch die Magnetpulse beeinflusst wird.

Die Wirksubstanzen und Wirkagenzien, die zu der aktivierten Eigenblut-Nosode gegeben werden, können aus gewöhnlichen Nosoden, gewöhnlichen homöopathischen Mitteln (beide hierin als "Wirkagenzien" bezeichnet) sowie allopathische Wirksubstanzen (sowohl in der Natur vorkommenden Wirksubstanzen und aus synthetischen Wirksubstanzen) ausgewählt sein. Die Auswahl richtet sich nach dem Krankheitsbild des Patienten und wird vom behandelnden Therapeuten bestimmt. Allopathische Wirksubstanzen werden lediglich in sehr geringen Mengen zugegeben, so dass die Transportkapazität des HSA in der Eigenblut-Nosode möglichst nicht überschritten wird.

Um nur einige wenige Beispiele zu nennen, können
als homöopathische Wirkagenzien
Hepar Comp (Fa. Heel),
Hypophysis / Stannum (Fa. Wala) und
Cuorum Injectopas (Fa. Pascoe);

als Nosoden
FSME C30,
Herpes Zoster Injeel (Fa. Heel),
Herpes vulgaris Injeel (Fa. Heel),
Sanukehl^{®} Staph D5/Staphyllococcus aureus D5 Ampullen und
EBV C30
und als allopathische Wirksubstanzen
alpha-Liponsäure, 600 mg
Folsäure Forte Hevert; in einer Menge von 20 mg
Pyrdoxinhydrochlorid (Vitamin B6); in einer Menge von 25 mg und
Regeneresen (RNA Frischzellen; Fa. Dyckerhoff Pharma)
zu der Eigenblut-Nosode gegeben werden.

Gewöhnlich werden bis zu vier Wirkagenzien oder Wirksubstanzen zu der Eigenblut-Nosode gegeben, bevor der Schritt der mechanischen Einwirkung vorgenommen wird. Es kann in speziellen Fällen aber auch nach jeder Zugabe eine mechanische Einwirkung sattfinden, bevor der weitere Aktivierungsschritt mit Licht vorgenommen wird.

Das Mischen der aktivierten Eigenblut-Nosode mit den Wirksubstanzen/ Wirkagenzien geschieht durch kurze mechanische Einwirkung, z. B. Erschütterung des Gefäßes ("Klopfen") oder, wie z. B. im Fall von Frischzellen, mehrmalige schnelle Translationsbewegung des Gefäßes ("Schütteln"). Bevorzugt werden die kurzen mechanischen Erschütterungen oder die kurze Translationsbewegung des Gefäßes etwa 20 bis etwa 150 Mal (je nach zugemischter bzw. zugemischtem Wirksubstanz/Wirkagens) vorgenommen.

Die so gewonnene Mischung oder modifizierte Eigenblut-Nosode wird auf die gleiche Weise wie oben für die Ausgangs-Eigenblut-Nosode beschrieben weiter durch Magnetpulse aktiviert und zusätzlich mit von LEDs erzeugtem sichtbarem Licht wechselnder Farbe, z.B. mit zwei oder mehr, bevorzugt drei oder vier oder mehr, mehr bevorzugt fünf oder sechs oder sogar mehr Farben, bestrahlt, im Allgemeinen über etwa 0,5 bis etwa 10 Minuten. z.B. über etwa 1 bis etwa 5 Minuten, und weiter vorzugsweise mit einer Leuchtstärke im Bereich von 3000 bis 5000 mcd, beispielsweise von 4000 mcd. Es ist aus der Komplementärmedizin bekannt, dass monochromatisches Licht im sichtbaren Bereich vielseitige Auswirkungen auf Blut hat, von denen eine die Steigerung der Bindungsfähigkeit von HSA ist. Das Licht kann zusätzlich zu den oben beschriebenen Magnetpulsen, die auch während der Lichteinstrahlung angewendet werden, noch durch ein den LEDs nachgeschaltetes Magnetfeld mit einer Magnetfeldstärke von etwa 50 µT geleitet werden, das ferner noch durch zusätzliche Magnetpulse z.B. mit einer Feldstärke von 20 µT mit Frequenzen z.B. im Bereich von 120 -140 Hz und von etwa 90 kHz moduliert werden kann. Eine Vorrichtung mit geeigneten LEDs, die auch einen Permanentmagneten und ein Magnetspule zur Erzeugung geeigneter permanenter und pulsierender Magnetfelder umfasst, ist unter dem Handelsnamen FW-Pen nach Prof.Schaack, CE, Helzel Messtechnik GmbH, Carl-Benz-Straße 9, D-24568 Kaltenkirchen, erhältlich.

Die vorstehend beschriebenen Schritte der Zugabe eines bzw. einer oder mehrerer Wirkagenzien/Wirksubstanzen können mit der anfänglich herstellten Eigenblut-Nosode bis zu sechs oder siebenmal, bevorzugt bis zu viermal, wiederholt werden.

In der wie oben beschrieben mit einer bzw. einem oder mehreren Wirksubstanzen/Wirkagenzien modifizierten und mit Magnetpulse und Licht aktivierten Eigenblut-Nosode liegen die Wirksubstanzen/Wirkagenzien (oder zumindest ein Teil der Wirkagenzien) in enger Assoziation mit HSA vor. Dies gewährleistet ihren guten Transport zu dem Ort, wo ihre Wirkung gewünscht wird.

Eine Vorrichtung zur Durchführung des vorstehend erläuterten erfindungsgemäßen Verfahrens umfasst:
a) ein verschließbares Gehäuse,
b) einen in dem Gehäuse angeordneten Probenhalter, der ein transparentes Gefäß halten kann, in dem die erste Mischung von Schritt a) oder b) hergestellt wird oder enthalten ist,
c) eine Einrichtung, die wiederholt eine mechanische Erschütterung und/oder Translationsbewegung des Gefäßes bewirken kann,
d) eine Magnetspule, die auf das Gefäß gerichtete Magnetpulse mit Frequenzen der Magnetfeldperioden im Bereich von etwa 0,01 bis etwa 20.0000 Hz und Feldstärken von maximal 50 µT erzeugen kann; und
e) eine Einrichtung, die LEDs umfasst, welche sichtbares Licht mit verschiedenen Farben erzeugen und das erzeugte Licht in das Gefäß einstrahlen lassen können.

Bei dem Gehäuse kann es sich um irgendein verschließbares Metall- oder Kunststoffgehäuse handeln.

Das transparente Gefäß kann ein z.B. entfernbares Septum aufweisen, durch das die erforderlichen Flüssigkeiten zur Herstellung der Nosode eingespritzt werden. Feststoffe können nach Entfernen des Septums eingeführt werden.

Der Probenhalter b) ist im Allgemeinen eine klammerartige Einrichtung, in die das Gefäß eingeklemmt wird. Er kann beweglich zum Ausführung einer Schwenk- oder Schüttelbewegung ausgelegt sein (eine Alternative der Einrichtung c)).

Im Allgemeinen ist auch eine mechanische Erschütterung des Gefäßes erforderlich. Dies kann z.B. durch eine hammerartige Klopfvorrichtung (andere Alternative der Einrichtung c)) bewirkt werden.

In einer anderen Ausführungsform sind der Probenhalter und eine Einrichtung, die sowohl für eine mechanische Erschütterung des Gefäßes sorgen als auch eine Translationsbewegung ausführen kann, einstückig ausgebildet. Hierbei handelt es sich z.B. um eine Probenhalter, der mit einer Hebevorrichtung verbunden ist, die z.B. mittels eines Elektromotors an einer stabförmigen Halterung hinauf- und hinunterbewegt werden kann. Das Hinunterbewegen kann mit einem Aufprallen auf eine Unterlage verbunden sein, wodurch das Gefäß und sein Inhalt mechanisch erschüttert werden.

Weiter enthält das Gefäß eine Metall-, bevorzugt Kupfer-Magnetspule, die beim Durchleiten von geeigneten elektrischem Strömen Magnetpulse mit Frequenzen der Magnetfeldperioden im Bereich von etwa 0,01 bis etwa 20.0000 Hz und Feldstärken von maximal 50 µT erzeugen kann. In einer bevorzugten Ausführungsform ist die Spule zylinderförmig um das Gefäß herum angeordnet und ist mit einer magnetisch durchlässigen Verkleidung versehen.

Ferner enthält das Gefäß LEDs, die sichtbares Licht mit mindestens zwei, bevorzugt mindestens drei oder vier, mehr bevorzugt fünf Farben oder sogar mehr sowie deren Mischfarben erzeugen und in das transparente Gefäß einstrahlen können. Zum Beispiel sind Bänder, die derartige LEDs umfassen, sind im Handel erhältlich.

Das Gefäß enthält ferner die für die genannten Einrichtungen erforderliche Stromversorgung sowie Einrichtungen zur Steuerung dieser Einrichtungen gegebenenfalls mittels einer elektronischen Steuerung durch eine programmierten oder programmierbaren Chip.

Auf der Außenseite kann das Gefäß z.B. einen Ein/Aus-Schalter, eine Anzeigevorrichtung, ein Zählwerk zum Zählen der durchgeführten Verfahren, eine Einrichtung zum Einstellen der Zahl der mechanischen Erdschütterungen oder Translations-Bewegungsvorgänge, und einen USB-Anschluss oder eine andere Einrichtung zum Einlesen von Daten aufweisen. Die Betriebssteuerung für diese Betriebseinrichtungen ist dann ebenfalls im Gehäuse enthalten.

Das Gefäß kann gegebenenfalls auch noch eine Permanentmagneten enthalten, der ein permanentes Magnetfeld gleich oder kleiner als 50 µT aufweist.

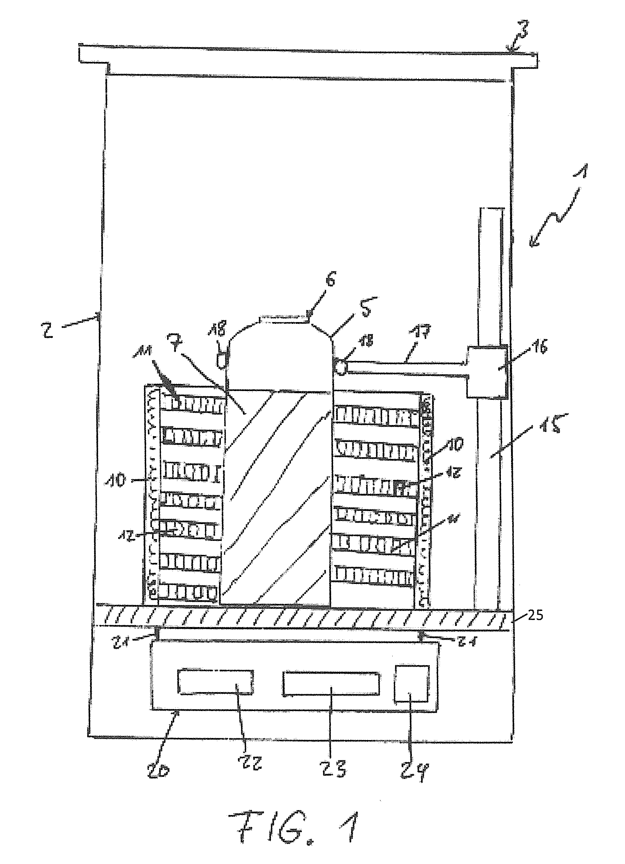

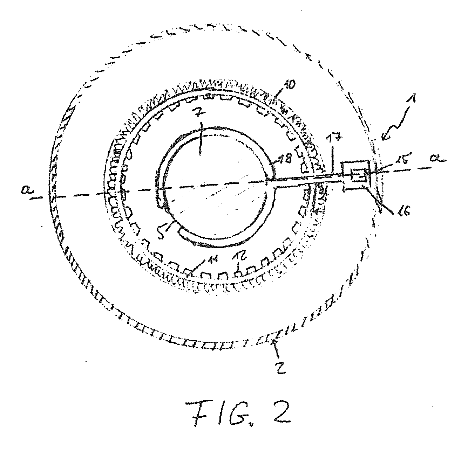

Figur 1 zeigt eine Schnittansicht entlang der Linie a-a von Fig. 2 einer Ausführungsform der erfindungsgemäßen Vorrichtung 1. In einem Gefäß 2, das mit einem Deckel 3 verschließbar ist, befindet sich ein transparentes Gefäß (z. B. ein Präparateglas) 5, das mit einem Septum 6 versehen ist, in einer Halterung 18, die über einen Arm 17 mit einer Hebevorrichtung 16 verbunden ist. Die Hebevorrichtung 16 kann mittels eines Elektromotors (nicht gezeigt) an der Halterung 15 hinauf und hinunterbewegt werden, wobei sie so weit nach unten bewegt werden kann, dass sie das Gefäß auf einer Zwischenbodenplatte 25 aufprallen lassen kann.

Das transparente Gefäß ist zylinderförmig von einer mit Kunststoff verkleideten Kupferspule 10 umgeben, die sich von der Zwischenbodenplatte 25 bis etwas zu einer Höhe unterhalb der Halterung 18, wenn sich dies im tiefsten Punkt befindet, erstreckt, bei oder unterhalb von sich die Oberfläche einer im Gefäß befindlichen Eigenblut-Nosode 7 befindet.

Auf der dem Gefäß zugewandte Seite der verkleideten Spule ist wendeiförmig ein LED-Band 11 mit LEDs 12 angebracht, die Licht mit fünf verschiedenen Wellenlängenbereichen im sichtbaren Bereich des elektromagnetischen Spektrums sowie Mischfarben daraus erzeugen können,

Unterhalb der Zwischenbodenplatte 25 befindet sich innerhalb einer Steuer- und Versorgungseinheit 20 eine an das Stromnetz anschließbare Stromversorgung 24, die über Kabel 21 die Strom an die Spule 10, die LEDs 12 und den Elektromotor für die Hebevorrichtung 16 liefert, eine elektronische Steuerungseinheit 23 zur Steuerung der Spule 10, der LEDs 12 und des Elektromotors für die Hebevorrichtung 16 sowie eine Betriebssteuerungseinheit 22 für die Steuerung der sich auf der Außenseite der Vorrichtung befindlichen Einrichtungen, nämlich einer Anzeigeeinrichtung 31, einer Einrichtung 32 zur Einstellung der Zahl der Bewegungen der Hebevorrichtung 16, eines Ein/Aus(On/Off)-Schalters 30" eines Zählers 33 für die Anzahl der Verfahren, die in der Vorrichtung durchgeführt wurden, und eines USB-Anschlusses 34.

### Beispiele

### Beispiel 1: Patient Herr H. aus Frankfurt, Jahrgang 1936

### A) Krankheitsbild

Bei diesem Patienten trat kurze Zeit nach einer FSME Impfung ein Ganzkörper-Ekzem mit starkem Juckreiz auf. Eine 6-wöchige medizinische Behandlung mit diversen allopathischen Mitteln, darunter Cortisonsalben, führte zu keinerlei Verbesserung.

### B) Nosoden-Herstellung am 09.06.2014

0,5 ml Blut des Pateinten wurden in einem Präparateglas 5 ml 70%iger ethanolischer Lösung gelöst und 5 Minuten magentisch mit einem vita-life®-R-Magnetstab (erhältlich von VITA LIFE HandelsgmbH, Gewerbepark1, 9220, Velden-Lind, Österreich) aktiviert. Danach wurden die nachstehend beschriebenen Mittel unter weiterer Magnetfeld-Aktivierung mit dem Magnetstab zugegeben.

| | |
|---|---|
| 30 Tropfen Q80 Fa. Calendula | 0,5 ml |
| 10 Globuli FSME Nosode C30 | 0,5ml |
| 1 Ampulle Hypophysis / Stannum | 1,0 ml |

Dann wurde die Mischung unter fortwährender Magnetfeld-Aktivierung durch 120-maliges Aufprallenlassen des Präparateglases auf einer Unterlage verschüttelt und 1 Minute mit einer FW-Pen nach Prof. Schaack, CE (Helzel Messtechnik GmbH, Carl-Benz-Straße 9, D-24568 Kaltenkirchen) bestrahlt.

Eine zweite Zugabe von Arzneimitteln geschah wie folgt:

| | |
|---|---|
| 1 Ampulle Histamin Injeel Heel | 2,0ml |
| 1 Ampulle Calcium Carbonicum Injeel Heel | 2,0ml |
| 1 Ampulle Glandula Thymi Injeel Heel | 2,0ml |
| 1 Ampulle Coenzympe Comp. | 2,0ml |
| | |
| Gesamtvolumen der zugesetzten Arzneimittel | 10 ml |

Dann wurde die modifizierte End-Eigenblut-Nosode unter weiterer fortwährender Magnetfeld-Aktivierung durch 100-maliges Aufprallenlassen des Präparateglases auf einer Unterlage verschüttelt und wiederum 1 Minute mit einer FW-Pen nach Prof. Schaack, CE (Helzel Messtechnik GmbH, Carl-Benz-Straße 9, D-24568 Kaltenkirchen) bestrahlt.

### C) Einnahme der modifizierten Eigenblut-Nosode

Die wie vorstehend beschrieben hergestellte modifizierte Eigenblut-Nosode wurde dem Patienten oral, 3 x tgl. 10 Tropfen, über die Dauer von 5 Tagen verbreicht. Die Nosode muss dabei 2 Minuten im Mund verbleiben (Einspeichelung), ohne dass geschluckt wird.

### D) Behandlungsergebnis gemäß Aussage des Patienten

Bereits nach wenigen Stunden hatte der Patient keinen Juckreiz mehr. Eine deutliche Abheilung des Ekzems begann bereits am zweiten Tag nach Nosodeneinnahme. Nach einer Woche hatte eine nahezu vollständige Ausheilung des Ekzems stattgefunden. Der Patient unterzieht sich derzeit einer Folgetherapie (biologische Regenerationsmedizin nach C. Klein).

### Beispiel 2: Patientin Frau M. aus Remshalden, Jahrgang 1942

### A) Krankheitsbild

Die Patientin hatte Asthma bronchiale und ein Lungenemphysem mit enormer Atmungseinschränkung aufgrund von vor 15 Jahren entnommenen rechten Lungensegmenten. Die rechte Lunge ist nur noch zu 1/3 vorhanden.
Ihre allopathische Medikation bestand aus:
- Viani mite 50/100
- Spiriva

### B) Nosode-Herstellung am 21.06.2014

0,5 ml Blut des Pateinten wurden in einem Präparateglas 5 ml 70%iger ethanolischer Lösung gelöst und 5 Minuten magentisch mit einem vita-life®-R-Magnetstab (erhältlich von VITA LIFE HandelsgmbH, Gewerbepark1, 9220, Velden-Lind, Österreich) aktiviert. Danach wurden die nachstehend beschriebenen Mittel unter weiterer Magnetfeld-Aktivierung mit dem Magnetstab zugegeben.

| | |
|---|---|
| 20 Tropfen ionisiert. Phosphorus D6 | 0,5ml |
| 15 Tropfen Atem Fa. Calendula | 0,5ml |
| 3 Ampullen N. Vagus GI D6 Wala | 3 x 2 ml = 6,0 ml |

Dann wurde die Mischung unter fortwährender Magnetfeld-Aktivierung durch 150-maliges Aufprallenlassen des Präparateglases auf einer Unterlage verschüttelt und 1 Minute mit einer FW-Pen nach Prof. Schaack, CE (Helzel Messtechnik GmbH, Carl-Benz-Straße 9, D-24568 Kaltenkirchen) bestrahlt.

Eine zweite Zugabe von Arzneimitteln geschah wie folgt:

| | |
|---|---|
| 2 Ampullen Injectio antiasthmatica Heel | 2 x 1 ml = 2,0ml |
| 2 Ampullen Medulla oblongata Injeel Heel | 2 x 2 ml = 4,0 ml |

Dann wurde diese erhaltene Mischung unter weiterer fortwährender Magnetfeld-Aktivierung durch 100-maliges Aufprallenlassen des Präparateglases auf einer Unterlage verschüttelt und wiederum 1 Minute mit einer FW-Pen nach Prof. Schaack, CE (Helzel Messtechnik GmbH, Carl-Benz-Straße 9, D-24568 Kaltenkirchen) bestrahlt.

Eine dritte Zugabe von Arzneimitteln geschah wie folgt:

| | |
|---|---|
| 2 Ampullen Bronchus suis Injeel Heel | 2x 2 ml = 4,0ml |
| 1 Ampulle Hypothalamus suis Injeel Heel | 2,0ml |

Dann wurde diese erhaltene Mischung unter weiterer fortwährender Magnetfeld-Aktivierung durch 150-maliges Aufprallenlassen des Präparateglases auf einer Unterlage verschüttelt und wiederum 1 Minute mit einer FW-Pen nach Prof. Schaack, CE (Helzel Messtechnik GmbH, Carl-Benz-Straße 9, D-24568 Kaltenkirchen) bestrahlt.

Eine vierte und letzte Zugabe von Arzneimitteln geschah wie folgt:

| | |
|---|---|
| 1 Ampulle Glandula Thymi suis Injeel Heel | 2,0ml |
| 1 Ampulle ATP Heel | 2,0ml |
| 12 Tropfen Lebensfreude Fa. Calendula | 0,5ml |
| 5 Tropfen Colchicum Hevert | 0,5ml |
| | |
| Gesamtvolumen der zugesetzten Arzneimittel | 24 ml |

Dann wurde die so erhaltene modifizierte End-Eigenblut-Nosode unter weiterer fortwährender Magnetfeld-Aktivierung durch 150-maliges Aufprallenlassen des Präparateglases auf einer Unterlage verschüttelt und wiederum 1 Minute mit einer FW-Pen nach Prof. Schaack, CE (Helzel Messtechnik GmbH, Carl-Benz-Straße 9, D-24568 Kaltenkirchen) bestrahlt.

### C) Einnahme der modifizierten Eigenblut-Nosode

Die wie vorstehend beschrieben hergestellte modifizierte Eigenblut-Nosode wurde dem Patienten oral, 5 x tgl. 10 Tropfen, über die Dauer von 14 Tagen verbreicht. Die Nosode muss dabei 2 Minuten im Mund verbleiben (Einspeichelung), ohne dass geschluckt wird.

Zur Nosode wurde 5 x tgl. 1 Hub Spenglersan Kolloid T von Meckel jeweils links und rechts in die Ellenbeuge gegeben.

### D) Behandlungsergebnis gemäß ärztlicher Beobachtung und Aussage der Patientin

Bereits 5 Minuten nach Einnahme der Nosode konnte eine deutliche Verbesserung der Atmung beobachtet werden. Nach 1 Stunde erklärte die Patientin, dass sie ohne sonstige Atemprobleme Treppen steigen könne, ohne pausieren zu müssen. Insgesamt fühle sie sich nahezu beschwerdefrei sowie leistungsfähiger.
Die Beschwerdefreiheit hielt in den letzten 14 Tagen an, laut Einschätzung der Patientin beträgt die Verbesserung der Atmung und der Leistungsfähigkeit ca. 80% mit Bezug auf den vorherigen Zustand.

Patientin unterzieht sich derzeit einer Folgetherapie (biologische Regenerationsmedizin nach C. Klein) mit Gabe einer Zweitnosode.

### Beispiel 3: Patient Herr F. aus Berlin, Jahrgang 1997

### A) Krankheitsbild

Der Patient litt seit 3 Monaten an Ohrenschmerzen mit Druckgefühl sowie an rechtsseitigen Schluckbeschwerden. Ein serologisches Bild lag vor. Das vom Hausarzt verordnete Antibiotikum führte zu keiner Verbesserung. Eine anschließende, entsprechende Dysbioselenkung wurde eingeleitet und durchgeführt.

### B) Nosoden-Herstellung am 21.05.2014

0,5 ml Blut des Patienten wurden in einem Präparateglas 5 ml 70%iger ethanolischer Lösung gelöst und 5 Minuten magentisch mit einem vita-life®-R-Magnetstab (erhältlich von VITA LIFE HandelsgmbH, Gewerbepark1, 9220, Velden-Lind, Österreich) aktiviert. Danach wurden die nachstehend beschriebenen Mittel unter weiterer Magnetfeld-Aktivierung mit dem Magnetstab zugegeben.

| | |
|---|---|
| 10 Tropfen EBV Nosode C30 | 0,5ml |
| 10 Tropfen Sanukehl Staphylokokken D6 | 0,5ml |
| 10 Tropfen ion. Fluor D6 | 0,5ml |

Dann wurde die Mischung unter fortwährender Magnetfeld-Aktivierung durch 100-maliges Aufprallenlassen des Präparateglases auf einer Unterlage verschüttelt und 1 Minute mit einer FW-Pen nach Prof. Schaack, CE (Helzel Messtechnik GmbH, Carl-Benz-Straße 9, D-24568 Kaltenkirchen) bestrahlt.

Eine zweite Zugabe von Arzneimitteln geschah wie folgt:

| | |
|---|---|
| 1 Ampulle Folsäure forte Hevert | 2,0ml |
| 1 Ampulle Hypophysis / Stannum | 1,0ml |
| 20 Tropfen Q80 Fa. Calendula | 0,5ml |

Dann wurde diese erhaltene Mischung unter weiterer fortwährender Magnetfeld-Aktivierung durch 100-maliges Aufprallenlassen des Präparateglases auf einer Unterlage verschüttelt und wiederum 1 Minute mit einer FW-Pen nach Prof. Schaack, CE (Helzel Messtechnik GmbH, Carl-Benz-Straße 9, D-24568 Kaltenkirchen) bestrahlt.

Eine dritte Zugabe von Arzneimitteln geschah wie folgt:

| | |
|---|---|
| 2 Ampullen Para-Benzochinon Fa. Heel | 4,0ml |
| 1 Ampulle N. Vagus GI D6 Wala | 2,0ml |

Dann wurde diese erhaltene Mischung unter weiterer fortwährender Magnetfeld-Aktivierung durch 100-maliges Aufprallenlassen des Präparateglases auf einer Unterlage verschüttelt und wiederum 1 Minute mit einer FW-Pen nach Prof. Schaack, CE (Helzel Messtechnik GmbH, Carl-Benz-Straße 9, D-24568 Kaltenkirchen) bestrahlt.

Eine vierte und letzte Zugabe von Arzneimitteln geschah wie folgt:

| | |
|---|---|
| 10 Tropfen Regenaplex 102 | 0,5ml |
| 10 Globuli Arnica C30 | 0,0 ml |
| | |
| Gesamtvolumen der zugesetzten Arzneimittel | 11,5ml |

Dann wurde die so erhaltene modifizierte Eigenblut-Nosode unter weiterer fortwährender Magnetfeld-Aktivierung durch 100-maliges Aufprallenlassen des Präparateglases auf einer Unterlage verschüttelt und wiederum 1 Minute mit einer FW-Pen nach Prof. Schaack, CE (Helzel Messtechnik GmbH, Carl-Benz-Straße 9, D-24568 Kaltenkirchen) bestrahlt und zum Schluss zum Erhalt der modifizierte End-Eigenblut-Nosode mit 705-igem Ethanol auf 25 ml aufgefüllt.

### C) Einnahme der modifizierten Eigenblut-Nosode

Die wie vorstehend beschrieben hergestellte modifizierte Eigenblut-Nosode wurde dem Patienten oral, 3 x tgl. 15 Tropfen, über die Dauer von 3 Tagen verbreicht. Die Nosode muss dabei 2 Minuten im Mund verbleiben(Einspeichelung), ohne dass geschluckt wird.

### D) Behandlungsergebnis gemäß Aussagen des Patienten

Bereits 5 Minuten nach Einnahme der Nosode beobachtete er ein sehr deutliches bis vollständiges Nachlassen des Druckgefühls auf den Ohren, ebenso ein Nachlassen der Schluckbeschwerden. Laut Aussage des Patienten waren ab diesem Zeitpunkt keine weiteren Beschwerden vorhanden.

Nach einigen Tagen stellte sich ein vermutlich Halswirbelsäulen-bedingter Tinnitus ein. Eine Überprüfung beim HNO ergab keinerlei Hörprobleme oder Entzündungen. Eine entsprechende weiterhin unterstützende Therapie-Empfehlung wurde dem Patienten vorgelegt. Es liegen keine weiteren Informationen zum heutigen Stand vor.

### Beispiel 4: Kleinkind, 2 1/2 Jahre

### A) Krankheitsbild

Seit 2 Jahren hatte das Kind andauernde Lähmungserscheinungen, anfänglich mit extremer Schläfrigkeit. Dies trat 1 Tag nach einer vom Kinderarzt durchgeführten 6-fach Impfung auf. Zuvor hatte sich das Kind normal entwickelt, normal geschlafen und gegessen. Seit dem Zeitpunkt der Impfung musste es zum Essen geweckt werden. Insgesamt war das Kind in der Entwicklung insbesondere auch der Füße zurückgeblieben. Es wies ein großes allgemeines Aggressionspotential (haute um sich und schrie) und eine hohe muskuläre Anspannung auf.

Von einem weiteren Kinderarzt wurden später Hypophysis / Stannum Globuli verordnet, woraufhin das Kleinkind aus der Schläfrigkeit erwachte und einen normalen Schlafrhythmus fand.

### B) Nosoden-Herstellung am 2.5.2014

1 Tropfen Blut des Kindes wurde in 1 ml physiologischer Kochsalzlösung (0,9%-ige NaCl-Lösung) gelöst und 5 Minuten magentisch mit einem vita-life®-R-Magnetstab (erhältlich von VITA LIFE HandelsgmbH, Gewerbepark1, 9220, Velden-Lind, Österreich) aktiviert. Danach wurden die nachstehend beschriebenen Mittel unter weiterer Magnetfeld-Aktivierung mit dem Magnetstab zugegeben.

| | |
|---|---|
| 5 Tropfen ionisiertes Fluor D6 | 0,5ml |
| 1 Ampulle Hypophysis / Stannum Fa. Wala | 1,0ml |

Dann wurde die Mischung unter fortwährender Magnetfeld-Aktivierung durch 100-maliges Aufprallenlassen des Präparateglases auf einer Unterlage verschüttelt und 1 Minute mit einer FW-Pen nach Prof. Schaack, CE (Helzel Messtechnik GmbH, Carl-Benz-Straße 9, D-24568 Kaltenkirchen) bestrahlt.

Eine zweite Zugabe von Arzneimitteln geschah wie folgt:

| | |
|---|---|
| 1 Tropfen Atem Elixier, Calendula Kräutergarten | 0,5ml |
| 1 Ampulle Folsäure forte Fa. Hevert | 2,0ml |

Dann wurde diese erhaltene Mischung unter weiterer fortwährender Magnetfeld-Aktivierung durch 100-maliges Aufprallenlassen des Präparateglases auf einer Unterlage verschüttelt und wiederum 1 Minute mit einer FW-Pen nach Prof. Schaack, CE (Helzel Messtechnik GmbH, Carl-Benz-Straße 9, D-24568 Kaltenkirchen) bestrahlt.

Eine dritte Zugabe von Arzneimitteln geschah wie folgt:

| | |
|---|---|
| 3 Tabletten Mercurius solubilis C6 | 0,0ml |
| 10 Tropfen ionisierter Phosphor D6 | 0,5ml |
| 10 Tropfen ionisiertes Magnesium D6 | 0,5ml |

Dann wurde diese erhaltene Mischung unter weiterer fortwährender Magnetfeld-Aktivierung durch 100-maliges Aufprallenlassen des Präparateglases auf einer Unterlage verschüttelt und wiederum 1 Minute mit einer FW-Pen nach Prof. Schaack, CE (Helzel Messtechnik GmbH, Carl-Benz-Straße 9, D-24568 Kaltenkirchen) bestrahlt.

Eine vierte Zugabe von Arzneimitteln geschah wie folgt:

| | |
|---|---|
| 1 Ampulle Cerebrum suis Injeel Fa. Heel | 2,0ml |
| 10 Tropfen Regenaplex N° 109 | 0,5ml |
| 10 Tropfen Regenaplex N° 112 | 0,5ml |
| | |
| Gesamtvolumen der zugesetzten Arzneimittel: | 8 ml |

Dann wurde die so erhaltene modifizierte Eigenblut-Nososde unter weiterer fortwährender Magnetfeld-Aktivierung durch 100-maliges Aufprallenlassen des Präparateglases auf einer Unterlage verschüttelt und wiederum 1 Minute mit einer FW-Pen nach Prof. Schaack, CE (Helzel Messtechnik GmbH, Carl-Benz-Straße 9, [ 24568 Kaltenkirchen) bestrahlt.

Zum Schluss wurden noch 2 Kapseln mit jeweils 500 mg L-Ornithin dazugegeben, wodurch die modifizierte End-Eigenblut-Nosode hergestellt wurde.

### C) Einnahme der modifizierten Eigenblut-Nosode

Die wie vorstehend beschrieben hergestellte modifizierte Eigenblut-Nosode wurde dem Patienten oral, 3 x tgl. 10 Tropfen, über die Dauer von 10 Tagen verbreicht. Die Nosode muss dabei 2 Minuten im Mund verbleiben (Einspeichelung), ohne dass geschluckt wird.

### D) Behandlungsergebnis gemäß Aussage der Mutter des Patienten

Nach Gabe der Nosode trat eine sofortige Verbesserung des Gemütszustandes ein. Das Kind zeige keine Aggressionen mehr, man konnte ein Nachlassen der Anspannung, eine weitere Verbesserung des Schlafes, einen Rückgang der Lähmungserscheinung der Füße bis hin zur normalen Entwicklung des Bewegungsapparates beobachten. Kind kann nunmehr alleine Treppen steigen und sich anziehen. Ferner spielt es seitdem mit anderen Kindern im Kindergarten, was zuvor nicht der Fall war. Insgesamt stellten sich eine normale Entwicklung und eine Verbesserung der Sprache ein.

Zur Nosodentherapie wurde eine Therapie-Empfehlung der biologischen Regenerationstherapie verordnet. Diese beinhaltete eine mineralische Therapie sowie eine Dysbioselenkung.

Die Mutter stellt in einem Brief fest:
" ...Mit dem Tag der ersten Einnahme merkte man deutliche Unterschiede zu vorher. Er hat keine unkontrollierten Wutausbrüche mehr!! Er ist viel aktiver, auch in der Entwicklung macht er deutliche Fortschritte. .... seine Kindergärtnerinnen bestätigen den Fortschritt. Sie sagen, es ist unglaublich, er wäre wie ausgewechselt!!! ....Er redet viel mehr und vor allem besser. Spielt mit anderen Kindern!! Er ist nicht mehr so in sich gekehrt......in der Entwicklung macht er extreme Fortschritte."

Alle hierin zitierten Druckschriften werden hierin mit ihrem gesamten Inhalt durch Bezugnahme aufgenommen.

## Patentansprüche

1. Verfahren zur Bindung einer Wirksubstanz oder eines Wirkagens an eine aktivierte Eigenblut-Nosode, umfassend:
a) Lösen von Blut eines Patienten in einem wässrigen oder wässrig/ethanolischen Medium oder Verreiben von Blut eines Patienten mit einem nach HAB für Globuli zugelassenen Trägerstoff zum Erhalt einer ersten Mischung;
b) Aktivieren der ersten Mischung durch Einwirkenlassen von Magnetpulse mit Frequenzen der Magnetfeldperioden innerhalb eines Bereichs von etwa 0,01 bis etwa 20.0000 Hz und Feldstärken von maximal 50 µT auf die erste Mischung zum Erhalt einer aktivierten ersten Mischung;
c) Zugabe einer bzw. eines oder mehrerer Wirksubstanzen und/oder Wirkagenzien zu der aktivierten ersten Mischung zum Erhalt einer zweiten Mischung;
d) Verschüttelung der zweiten Mischung durch mechanische Einwirkung, wobei die Schritte c) und d) und fortwährender Einwirkung der Magnetpulse vorgenommen werden und wobei die Schritte c) und d) einmal oder mehrmals wiederholt werden können, wodurch man eine verschüttelte zweite Mischung erhält; und
e) Aktivieren der verschüttelte zweiten Mischung durch weiteres fortwährendes Einwirkenlassen der Magnetpulse und Einstrahlen sichtbaren, von LEDs erzeugten Lichts wechselnder Farbe in die verschüttelte zweite Mischung, wodurch eine Steigerung der Bindungsfähigkeit des Human-Serumalbumin (HSA) in dem Blut an die Wirksubstanz(en) und/oder an zumindest Teile des Wirkagens bzw. der Wirkagenzien bewirkt wird und eine modifizierte Eigenblut-Nosode erhalten wird.

2. Verfahren nach Anspruch 1, **dadurch gekennzeichnet, dass** die Menge des Bluts 1 Tropfen bis 2 ml ist und das Blut in 1 bis 10 ml 70%igem wässrigem Ethanol gelöst wird.

3. Verfahren nach Anspruch 1 oder 2, **dadurch gekennzeichnet, dass** die Wirksubstanz(en) oder das Wirkagens oder die Wirkagenzien aus einem oder mehreren Mitgliedern der Arzneimittelgruppe bestehend aus Nosoden, homöopathischen Arzneimitteln und allopathischen Arzneimitteln ausgewählt sind.

4. Verfahren nach irgendeinem der Ansprüche 1 bis 3, **dadurch gekennzeichnet, dass** man die mechanische Einwirkung im Schritt e) etwa 20 bis etwa 200, bevorzugt etwa 50 bis etwa 150 Mal aufeinander folgend durchgeführt wird.

5. Verfahren nach irgendeinem der Ansprüche 1 bis 4, **dadurch gekennzeichnet, dass** die Aktivierung durch Einwirkenlassen von Magnetpulsen das Einwirkenlassen von Nadelpuls-Paketen mit dazwischen liegenden Pausen umfasst.

6. Verfahren nach irgendeinem der Ansprüche 1 bis 5, **dadurch gekennzeichnet, dass** das sichtbare, von LEDs erzeugte Licht wechselnder Farbe ein Leuchtstärke von etwa 4000 mcd besitzt und etwa 1 bis etwa 5 Minuten lang eingestrahlt wird.

7. Verfahren nach irgendeinem der Ansprüche 1 bis 6, **dadurch gekennzeichnet, dass** das sichtbare, von LEDs erzeugte Licht wechselnder Farbe ferner durch ein den LEDs nachgeschaltetes Magnetfeld mit einer Magnetfeldstärke von etwa 50 µT tritt, das zusätzlich durch Magnetpulse mit einer Feldstärke von 20 µT mit Frequenzen im Bereich von 120 -140 Hz und etwa 90 kHz moduliert wird.

8. Vorrichtung zur Durchführung des Verfahrens nach einem der Ansprüche 1 bis 7, umfassend:
1) ein verschließbares Gehäuse (2),
2) einen in dem Gehäuse angeordneten Probenhalter (18), der ein transparentes Gefäß (5) halten kann, in dem die erste Mischung (7) von Schritt a) oder b) hergestellt wird oder enthalten ist,
3) eine Einrichtung (15, 16), die wiederholt eine mechanische Erschütterung und/oder Translationsbewegung des Gefäßes bewirken kann,
4) eine Magnetspule (10), die auf das Gefäß gerichtete Magnetpulse mit Frequenzen der Magnetfeldperioden im Bereich von etwa 0,01 bis etwa 20.0000 Hz und Feldstärken von maximal 50 µT erzeugen kann; und
5) eine Einrichtung (11), die LEDs (12) umfasst, welche sichtbares Licht mit mindestens zwei Farben erzeugen und das erzeugte Licht in das Gefäß (6) einstrahlen lassen können.

9. Vorrichtung nach Anspruch 8, **dadurch gekennzeichnet, dass** die Halterung 18 und die Einrichtung (15, 16), die wiederholt eine mechanische Erschütterung und/oder Translationsbewegung des Gefäßes bewirken kann, über einen Arm (17) verbunden sind.

10. Vorrichtung nach Anspruch 8 oder 9, **dadurch gekennzeichnet, dass** die Magnetspule 10 zylinderförmig um das Gefäß 5 herum angeordnet ist.

11. Vorrichtung nach einem der Ansprüche 8 bis 10, **dadurch gekennzeichnet, dass** sich die LEDs (12) in oder auf einem LED-Band (11) befinden, das wendelförmig auf der dem Gefäß zugewandten Seite der Spule 10 angeordnet ist.

12. Modifizierte Eigenblut-Nosode, herstellbar gemäß dem Verfahren von irgendeinem der Ansprüche 1 bis 7.
